Europäisches Patentamt

European Patent Office

Office européen des brevets

(11) Veröffentlichungsnummer: **0 292 640**

**A1**

(12) # EUROPÄISCHE PATENTANMELDUNG

(21) Anmeldenummer: 88101346.0

(51) Int. Cl.⁴: **A61M 16/08**

(22) Anmeldetag: 30.01.88

Die Bezeichnung der Erfindung wurde geändert (Richtlinien für die Prüfung im EPA, A-III, 7.3).

(30) Priorität: 31.01.87 DE 3702917

(43) Veröffentlichungstag der Anmeldung:
30.11.88 Patentblatt 88/48

(84) Benannte Vertragsstaaten:
AT BE CH ES FR GB GR IT LI LU NL SE

(71) Anmelder: **THYSSEN PLASTIK ANGER KG**
**Anzinger Strasse 1**
**D-8000 München 80(DE)**

(72) Erfinder: **Schwarzensteiner, Hermann**
**Girletweg 5**
**D-8447 Windberg(DE)**

(54) **Anschlusskopf für Beatmungsgeräte.**

(57) Die Erfindung bezieht sich auf einen Anschlußkopf (1) in der Atemluftversorgung von Patienten mit Luftzu- und -abfuhranschlüssen (1b,1c) und ggf. einem Adapter (2) für eine Trachealkanüle, bei dem mindestens einer der Schlauchanschlüsse (1b,1c) am Anschlußstutzen (3,4) drehbar ausgebildet ist, wobei auch ein Kugelgelenk oder dergl. vorgesehen sein kann und die gegeneinander drehbaren Teile des Anschlußkopfes (1) und der Schlauchanschlüsse (1b,1c) geringe Auflageflächen (F3,F4) miteinander aufweisen.

Fig. 1

Fig. 3

EP 0 292 640 A1

## ANSCHLUSSKOPF

Die Erfindung bezieht sich auf einen Anschlußkopf in der Atemluftversorgung von Patienten mit Luftzu- und -abfuhranschlüssen und ggf. einem Adapter für eine Trachealkanüle.

Anschlußköpfe für die Atemluftversorgung von Patienten müssen mehreren Anforderungen genügen. So hat ein solcher Anschlußkopf den in der Medizin üblichen Sicherheitsanforderungen zu genügen, geringe Toleranzen aufzuweisen und muß so ausgebildet sein, daß seine Luftzu- und abfuhrstutzen nicht unbeabsichtigt verschlossen werden können. Er soll außerdem einen geringen Totraum aufweisen usw. Diese Anforderungen erfüllt der Anschlußkopf gemäß der DE-OS 33 39 988 weitgehend, indem der zentrale Hohlraum dieses Anschlußkopfes zwischen den Luftzu- und -abfuhrstutzen Abflachungen und der Adapter eine ebene Stirnfläche zur Ver ringerung des Totraumes aufweist. Die weitergehende Forderung der medizinischen Praxis, vor allem bei Frühgeburten, die sich für eine gwisse Zeit in einem Brutkasten aufhalten müssen, die Patienten laufend zu bewegen, insbesondere zu drehen, ohne daß sich dabei Probleme mit der Luftversorgung des Patienten ergeben, kann damit jedoch nicht gelöst werden. Die Anschlußschläuche des Anschlußkopfes haben sich während des Betriebes als zu starr erwiesen, um die Bewegungen des Patienten mitmachen zu können. Vielmehr ergeben sich Spannungen in den Schläuchen bei Drehung des Patienten, wodurch der Anschlußkopf während der Drehung aus dessen Nase bzw. Mund rutschen kann. Aus diesem Grunde werden diese Anschlußschläuche vor der Drehung des Patienten üblicherweise ab- und nach erfolgter Drehung wieder aufgesteckt.

Es liegt auf der Hand, daß ein solches Vorgehen in der täglichen klinischen Praxis nicht nur umständlich ist, sondern auch erhebliche Gefahren für den Patienten mit sich bringt.

Der Erfindung liegt daher die Aufgabe zugrunde, einen Anschlußkopf zu besitzen, der bei Drehung des Patienten die Spannung der angeschlossenen Schläuche auffängt und somit das Herausrutschen der Kanüle aus der Nase bzw. dem Mund des Patienten verhindert und dem Personal die Handhabung der Atemluftversorgung erleichtert.

Erfindungsgemäß wird dies dadurch erreicht, daß mindestens einer der Schlauchanschlüsse am Anschlußstutzen drehbar ausgebildet ist.

Vorteilhafterweise sind die Schlauchanschlüsse der in verschiedenen Ebenen an- bzw. abgehende Schläuche jeweils axial drehbar.

Vorteilhafterweise ist zwischen Schlauchanschluß und Anschlußstutzen ein Kugelgelenk vorgesehen.

Vorteilhafterweise stehen dieKupplungsebenen in verschiedenen Winkeln aufeinander.

Vorteilhafterweise weisen die gegeneinander drehbaren Teile des Anschlußkopfes und der Schlauchanschlüsse geringe Auflagenflächen miteinander auf.

Ein solcherart ausgebildeter Anschlukopf weist den Vorteil äußerst geringer Spannung in den angeschlossenen Schläuchen bei Drehung des Patienten auf, wobei Toleranzen weitgehend überhaupt keine Rolle mehr spielen. Ein weiterer Vorteil ist, daß das Personal entlastet wird und somit keine Fehler mehr bei der Atemluftversorgung der Patienten begehen kann.

Die Erfindung ist nachstehend anhand eines in den Abbildungen dargestellten Ausführungsbeispieles näher erläutert.

Es zeigt:

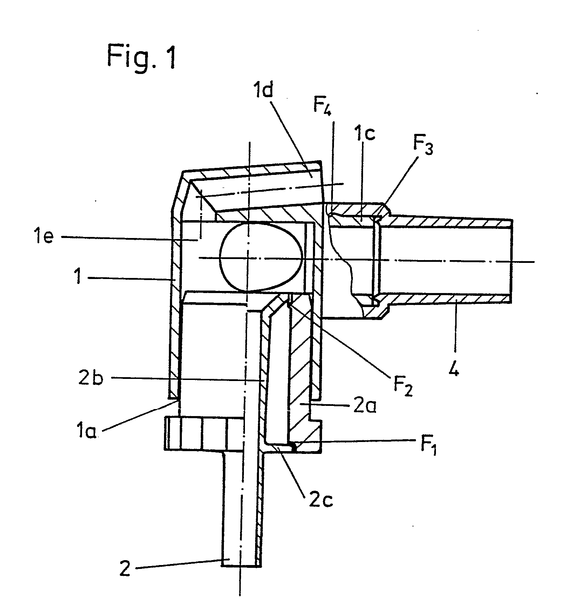

Figur 1 einen Anschlußkopf im Schnitt, mit Teilschnitt im Luftanschluß,

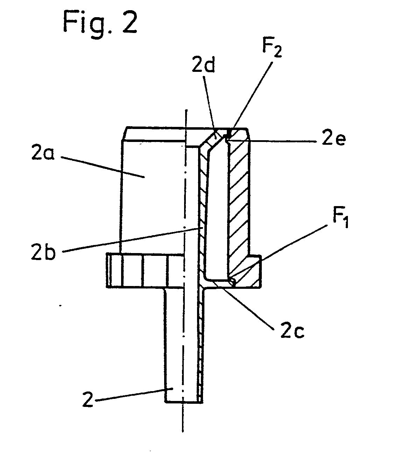

Figur 2 den Adapter des Anschlußkopfes im Halbschnitt,

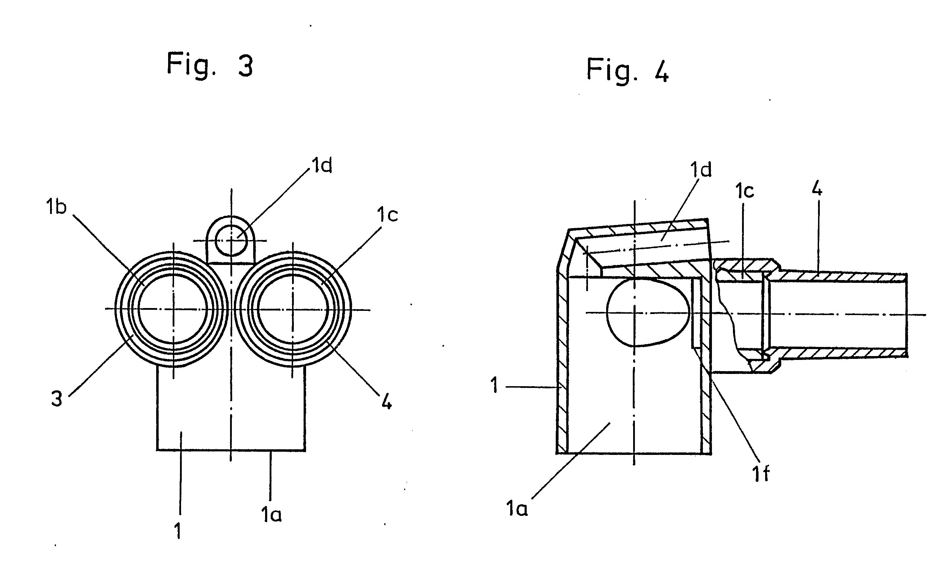

Figur 3 den Anschlußkopf ohne Adapter, und

Figur 4 einen Schnitt in Richtung A-B durch Figur 3.

Der in den Figuren dargestellte Anschlußkopf 1 ist mit einem Anschluß 1a für den Tubusadapter der Trachealkanüle, einem Anschluß 1b für die Luftzufuhr, einem Anchluß 1c für die Luftabfuhr und einem Anschluß 1d für eine Druckmeßsonde ausgerüstet. Diese Anschlüsse sind in unterschiedlichen Winkeln zueinander vorgesehen. Deren Lage zueinander ist durch die Verhältnisse im Brutkasten oder dergl. bedingt.

Der Anschlußkopf 1 weist darüber hinaus einen äußerst klein gehaltenen Totraum 1e auf, von dem die einzelnen Anschlüsse abgehen. Mit 1f ist ein Anschlag im Tubusanschluß 1a, für die Schnapphülse 2a des Tubusadapters 2 bezeichnet. Dieser Tubusadapter 2 besteht aus zwei Teilen, nämlich der Schnapphülse 2a und dem Anschlußkörper 2b, die ineinander geschoben und miteinander verrastet sind. Die Auflageflächen F1 und F2 der beiden Teile 2a und 2b sind so gering bemessen, daß der Anschlußkörper 2b sich leicht in der Schnapphülse 2a drehen läßt. Die notwendige Dichtheit beider Teile zueinander wird dadurch erreicht, daß eine leichte Bundvorspannung in der Rastung an den Flächen F1 und F2 vorgesehen ist. Beim Einschieben des Anschlußkörpers 2b in die Schnapphülse 2a legt sich diese mit ihrem unteren Bund 2c an der entsprechend ausgebildeten Auflagefläche F1 an, während durch - leichtes Nachdrücken bei der Montage der - obere, schräg ausgebildete Bund 2d in den Vorsprung 2e in der Schnapphülse 2a einrastet und unter Vorspannung sich an die Fläche F2 und den Vorsprung 2e anlegt. Somit ist bei leichter Drehbarkeit des Anschlußkörpers 2b in der Schnapphülse 2a ein Herausrutschen verhindert und die notwendige Dichtheit gewährleistet.

In den Anschlüssen für die Luftzu- und -abfuhr 1b und 1c sind entsprechende Auflageflächen F3, F4 für den Luftabfuhrstutzen 4 vorgesehen, während die Flächen für den Luftzufuhrstutzen 3 in der Abbildung nicht dargestellt sind. Auch diese Luftzu- und abfuhrstutzen 3 und 4 sind somit leicht in den Anschlüssen 1b und 1c drehbar.

Ein Druckmeßanschluß 1d ist oberhalb des Totraumes 1e vorgesehen, in den bei Bedarf ein entsprechender Instrumentenanschluß eingesteckt werden kann, bzw. falls nicht notwendig, kann dieser Anschluß auch mit einem Stöpsel verschlossen werden.

Wird der Patient gedreht, bewegt sich die in Mund und/oder Nase eingeführte Kanüle mit dem Patienten, ohne daß sie herausrutscht, bzw. sich von den Schläuchen löst, da sowohl die Schlauchenden Drehung mitmachen, indem sie einzeln oder zusammen um ihre Achse, bzw. jeweiligen Achsen, im Anschlußkopf drehbar sind, als auch diese Drehbarkeit in Verbindung mit der Elastizität der Schläuche die Bewegungsfähigkeit des Patienten nicht einengt, Fehler also weitgehend von vornherein ausgeschaltet sind.

Der erfindungsgemäß vorgeschlagene Anschlußkopf kann selbstverständlich auch auf anderen Gebieten mit Vorteil eingesetzt werden und ist auch nicht auf den Einbau von axial drehbaren Verbindungen zwischen Schläuchen und Anschlußkopf, bzw. Kugelgelenken beschränkt.

## Ansprüche

1. Anschlußkopf in der Atemluftversorgung von Patienten mit Luftzu- und -abfuhranschlüssen und ggf. einem Adapter für eine Trachealkanüle, **dadurch gekennzeichnet,** daß die Schlauchanschlüsse (1a, 1b, 1c, 1d) der in verschiedenen Ebenen an- bzw. abgehende Schläuche jeweils axial drehbar sind.

2. Anschlußkopf nach Anspruch 1, **dadurch gekennzeichnet,** daß zwischen Schlauchanschluß und Anschlußstutzen (1) ein Kugelgelenk vorgesehen ist.

3. Anschlußkopf nach Anspruch 1 und 2, **dadurch gekennzeichnet,** daß die gegeneinander drehbaren Teile des Anschlußkopfes (1) und der Schlauchanschlüsse (1a, 1b, 1c, 1d) geringe Auflageflächen miteinander aufweisen.

## Fig. 1

THYSSEN PLASTIK ANGER KG
Postfach 800140, Anzinger Straße 1
8000 München 80

0 292 640

# Fig. 2

THYSSEN PLASTIK ANGER KG
Postfach 800140, Anzinger Straße 1
8000 München 80

0 292 640

## Fig. 3

## Fig. 4

# EUROPÄISCHER RECHERCHENBERICHT

Nummer der Anmeldung

## EINSCHLÄGIGE DOKUMENTE

EP 88101346.0

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB - A - 1 396 098 (MED. & IND. EDQU.)<br><br>* Fig. 2; Seite 2, Zeilen 23-29,60-98 *<br><br>-- | 1 | A 61 M 16/08 |
| A | DE - A1 - 3 401 924 (DRÄGERWERK)<br><br>* Fig. 1,2; Seite 5, Zeilen 9-11,24-27; Seite 6, Zeilen 6-10,17-22; Patentanspruch 1 *<br><br>-- | 1 | |
| A | FR - A - 2 151 782 (FA.W. RÜSCH)<br><br>* Fig.; Seite 3, Zeilen 24-27; Seite 4, Zeilen 5-10; Patentansprüche1,6 *<br><br>& DE-U1-7 133 753<br><br>-- | 1 | |
| A | US - A - 4 152 017 (H.J.BRAMSON)<br><br>* Gesamt; insbesondere Spalte 3, Zeilen 2-6 *<br><br>-- | 1,3 | RECHERCHIERTE SACHGEBIETE (Int. Cl.4)<br><br>A 61 M 16/00 |
| D,A | DE - A1 - 3 339 988 (ANNEDORE KINNLE MED.)<br><br>* Fig. 1-4; Patentansprüche 1-3 *<br><br>---- | 1 | |

Der vorliegende Recherchenbericht wurde für alle Patentansprüche erstellt.

| Recherchenort | Abschlußdatum der Recherche | Prüfer |
|---|---|---|
| WIEN | 27-06-1988 | LUDWIG |